# EUROPEAN PATENT SPECIFICATION

(11) **EP 4 494 564 B1**
(45) Date of publication and mention of the grant of the patent: **14.10.2026**
(21) Application number: 23186133.7
(22) Date of filing: 18.07.2023
(51) Int. Cl.: A61B 6/04, A61B 5/00, G06T 7/73

(54) **METHOD FOR A POSITIONING CONTROL OF AN OBJECT UNDER INVESTIGATION BEFORE ACQUIRING A PROJECTIVE X-RAY IMAGE**
VERFAHREN ZUR POSITIONSSTEUERUNG EINES UNTERSUCHTEN OBJEKTS VOR DER ERFASSUNG EINES PROJEKTIVEN RÖNTGENBILDES
PROCÉDÉ DE COMMANDE DE POSITIONNEMENT D'UN OBJET EXAMINÉ AVANT L'ACQUISITION D'UNE IMAGE RADIOGRAPHIQUE PROJECTIVE

(43) Date of publication of application: 22.01.2025
(73) Proprietor: Siemens Healthineers AG, 91301 Forchheim (DE)
(72) Inventor: Biniazan, Ramyar, 90402 Nürnberg (DE); Fieselmann, Andreas, 91052 Erlangen (DE); Herbst, Magdalena, 91056 Erlangen (DE); Sutter, Sven-Martin, 91074 Herzogenaurach (DE)
(74) Representative: Siemens Healthineers Patent Attorneys

(56) References cited:
- US-A1- 2018 116 613
- US-A1- 2020 375 546
- US-A1- 2023 157 660

## Description

The invention relates to a computer-implemented method for a positioning control of an object under investigation before acquiring a projective X-ray image of a region of interest, a computer-implemented method to create a set of reference object models for at least one examination pose and at least one region of interest, a medical system, a computer program and a computer-readable medium, which provide an evaluation of an examination pose before acquiring a projective X-ray image.

In X-ray imaging, projective X-ray images are common for many imaging tasks. In a projective X-ray geometry, structures may overlap. X-rays are emitted by an X-ray source, partially absorbed by the tissue and bones of the object under investigation, and, finally, the remainder of the X-rays is registered by the X-ray detector. As output, an X-ray image can be displayed.

Measurements of length and angles are frequently made in musculoskeletal (MSK) X-ray examinations to assess orthopedic diseases. The accuracy of the measurement depends on the quality of the acquired image. Various factors that degrade image quality exist. For example, non-optimal patient positioning, especially relative to the X-ray source and the X-ray detector, can lead to an overlap of structures in the projection image and thereby incorrect measurement values.

Radiographers are well trained to assure best possible patient positioning such that the image can be used for the indication for which it was acquired. Physical disabilities of the patient must be taken into account when finding the most suitable positioning for this individual patient. The level of expertise of radiographers can vary. Therefore, the capability to find the best possible patient position can vary and depend on the radiographer.

Up to now patient positioning is normally assessed by the radiographer. Few automated software solutions exist for dedicated applications (e. g. chest x-ray exam) that provide feedback to the radiographer after the exam has been acquired. So far, no automated solution exists that can assess the patient positioning before the x-rays are released.

The technical problem to be overcome by this invention is that the image quality, especially with respect to the patient positioning, depends on the expertise of the radiographer acquiring the exam or rather the X-ray image.

The US 2023/0157660 A1 discloses a method for performing an automated scan preparation.

The US 2018/0116613 A1 discloses a positioning guidance for image acquisition.

The US 2020/0375546 A1 discloses a method for providing an indication for a recommended movement of a patient based on 3D surface map associated with the patient and a desired pose.

It is an object of the invention to provide a method which evaluates the patient positioning before acquiring the X-ray image by technical means.

According to the invention, the object is achieved by the features of the accompanying claims, in particular of a method and the respective computer program product and system. Advantageous aspects, features and embodiments are described in the dependent claims and in the following description together with advantages.

Independent of the grammatical term usage, individuals with male, female or other gender identities are included within the term.

The invention relates to a computer-implemented method for a positioning control of an object under investigation before acquiring a projective X-ray image of a region of interest, comprising:
- receiving an object information of the object under investigation in an examination pose,
- retrieving an object model relating to the examination pose and the region of interest,
- adapting the object model to the object under investigation based on the object information, and
- evaluating the examination pose based on the adapted object model, wherein the adapted object model is defined by object model parameters, wherein a reference object model is selected based on the object model parameters from a plurality of reference object models with assigned positioning deviations compared to a standard examination pose.

The computer-implemented method according to the invention is used to control or to check a positioning of an object under investigation before acquiring a projective X-ray image of a region of interest. Typically, the object under investigation is a patient.

The method comprises the steps of receiving, retrieving, adapting, and evaluating, preferably in the given order. An object information of the object under investigation in an examination pose is received. An object model relating to the examination pose and the region of interest is retrieved, e.g. from a database. The object model is adapted to the object under investigation based on the object information. The examination pose is evaluated based on the adapted object model.

In order to be able to use this method, at least one object model, preferably a plurality of object models, are made available. The inventors propose to create a number of reference object models based on an avatar-based simulation phase.

This step can be required to be done only once per measurement application. Results from this step can be used in the steps of adapting the object model to the object under investigation, the assessment or evaluation based on the matched avatar or adapted object model, and/or a recommendation of alternative positioning based on data of individual patients or individual objects under investigation.

The aim of the avatar-based simulation phase is to explore the relationship between patient positioning as the patient is represented by an avatar or by the object model and its impact on the measurement result. The results of simulation phase provide a relationship between the positioning of the object under investigation, which can be represented by the object model, and the impact on the measurement result, especially based on an acquired X-ray image of the object under investigation which is positioned in that specific position and/or pose.

Preferably, different object models are made available. A specific object model can be selected from the plurality of object models. The plurality of object models can include object models of young, old, normal, obese, slim, small, large or medium sized patients. The object under investigation is preferably a human patient. Anatomical models of the relevant anatomy, e.g. based on CT volumes, can be used to refine the object model. In a preferred embodiment, a motion model for the object model can be required in addition to the anatomical model. The motion model can provide a model regarding realistic motion of bones. The object model includes at least one landmark, especially a plurality of relevant landmarks, in 3D for the corresponding measurement task or examination. In a preferred embodiment, the object model must include relevant landmarks in 3D for the corresponding measurement task or examination. The imaging process is modelled by a forward projector for the landmarks in X-ray geometry. The landmarks of the object model are projected in X-ray geometry into an imaging plane. The anatomical model as well as the motion model are used to modify the object model in order to provide a reference object model wherein the patient position parameters **p** can be used to characterize the reference object model and/or the object model.

For the measurement task, which could be indicated by index j, but is omitted here in most formulas for better readability, relevant patient position parameters **p** must be defined. These comprise the patient posture, the patient orientation relative to the imaging setup and the geometry of the imaging system.

A reference position **p**^{ref} needs to be defined: ${p}^{ref} = {\left[{{p}_{1}}^{ref},{{p}_{2}}^{ref}…\right]}^{T} .$

For this reference position, the projected 2D landmarks **l**^{ref} are obtained by applying the forward projection to the 3D landmarks **l**^{vol} of the object model at position **p**^{ref}. From **p**^{ref}, the reference measurement m^{ref} is calculated using the function m^{ref} = M^{j} (**p**^{ref}) where M^{j} () describes the measurement process for a particular measurement or examination. The reference position can characterize the pose and/or the position of the reference object model, especially in a standard examination pose. The position can characterize an examination pose which can be different to the standard examination pose.

Equally, measurements mⁱ for positions **p**ⁱ covering the parameter space are obtained. The deviation ${d}^{i} = {m}^{ref} - {m}^{i} = M \left({p}^{ref}\right) - M \left({p}^{i}\right) = D \left({p}^{i}\right)$ of the measurements mⁱ to the reference measurement m^{ref} can be given as relative or absolute deviation. D() is a function depending on **p**ⁱ that computes the deviation for a pre-defined **p**^{ref}. The values dⁱ corresponding to **p**ⁱ are stored in a database. The values dⁱ can be given as absolute or relative values.

By receiving an object information of the object under investigation in an examination pose, object information is provided e.g. based on information acquired by an external source or sensor, or the X-ray detector in combination with the X-ray source, especially a pre-shot X-ray image acquisition. The object information can be a depth image of the object under investigation. The object information can be determined based on an image of the object under investigation. The object under investigation is positioned preferably according to the examination pose in connection with the X-ray system. A specific examination pose is assigned to a specific X-ray examination. An examination pose can describe e.g. a standing, sitting or lying pose. The examination pose can include instructions for angles or rotations of body parts.

According to an aspect of the invention, the object information is determined based on an image of the surface of the object under examination. The image of the surface can be acquired by a sensor or an external source. The external source or the sensor that records the external appearance of the patient, especially not recording the interior of the object under investigation, can include:
- an RGB camera,
- a depth camera,
- a structured light camera,
- an infrared camera, and/or
- a light detection and ranging (LiDAR) camera.

The external source or the sensor can be positioned at the medical system, at the imaging device or at a different location in the examination room.

The object information can be determined e.g. based on a landmark detection method. The object information can be determined e.g. based on a detection of the outline of the object under investigation. The object information can comprise a parameter relating to the size and/or weight of the object under investigation. The size can describe the full body length, or a measure of a body part or a body region. The object information can comprise the outer shape of the object under examination, especially the outer shape which can be observed from the position of the X-ray source. The outer shape can be made available in a grid representation comprising the depth information of at least a part of the object under examination.

According to an aspect of the invention, the object information is determined based on an X-ray image. The X-ray image can be a low dose X-ray pre-shot image. The low dose X-ray pre-shot image can show some information of the interior of the object under investigation but with less detail compared to a full X-ray image. An X-ray pre-shot could be used to acquire an image, in this case, the image comprises information of the internal appearance of body of the object under examination. The X-ray image can show details in a two-dimensional plane like the outline as well as some information about the internal structures of the object under investigation. The internal structures can comprise information about dense objects like bones, about soft tissue and/or about air volumes. The X-ray image provides two-dimensional information displayed by gray values.

By retrieving an object model relating to the examination pose and the region of interest, the object model is selected according to the examination pose and the region of interest and the object model made available to the system.

In a next step, the avatar can be matched to object under investigation. This means that the object model can be adapted to the object under investigation based on the object information. The object model can be called an avatar. The object model can be adapted or modified based on the object information. The object information can comprise parameters like dimensions of anatomical structures or dimensions of the outer shape of the object under investigation.

Data from external sources can be used to adapt the parameters of the avatar to the real patient. The object information can be acquired by external sources like a camera. The object information can comprise parameters which describe at least the outer shape of the object under investigation, respectively the patient. The object information is used to adapt the object model to the object under investigation. Preferably, the object information comprises a set of parameters which describe the object under investigation as well as the object model. A parameter could be for example the width of the chest of the object under investigation and the object model, respectively. The object information is used to adapt the object model in a way that at least the outer shape of the object is represented by the adapted object model.

To match the object model to the object under investigation, one has to find the correct surface transformation between surface of the object model and the object under investigation. Various methods for this task have been proposed in literature and could be used for this task. The work by Shuai Lin, Yu-Kun Lai, Ralph R. Martin, Shiyao Jin, Zhi-Quan Cheng, Color-aware surface registration, Computers & Graphics, Volume 58, 2016, Pages 31-42, https://doi.org/10.1016/j.cag.2016.05.007*,* for example, introduced a novel color-aware registration method for rigid and non-rigid registration in which the inputs from depth cameras will provide initial alignment and deformable registration steps will improve it for having a surface with high quality textures. It is known that this step has a certain range of accuracy due to individual characteristics of the object under investigation. Nevertheless, one can use this step or this method to obtain a statistical estimation with a certain confidence range.

According to an aspect of the invention, the object model is adapted by using a surface transformation function based on the object information. The surface or the outer shape of the object model can be adapted to the surface or the outer shape of the object under investigation. The object information can be used to bring the surface of the object model in conformity with the surface of the object under investigation, at least to a certain degree.

Surface transformations of a human shape based on depth images are well known. These transformations involve the analysis and manipulation of depth images to extract meaningful information about the shape and structure of human bodies. By understanding the surface properties of a human shape, a gesture recognition and a pose estimation can be performed based on (depth) images.

Depth images capture the distance information of objects in a scene, typically represented as a two-dimensional matrix of depth values. These depth values provide a measurement of the distance between the camera and the observed object's surface. For human bodies, depth images can offer valuable insights into the shape, posture, and movements of individuals, as they can capture the three-dimensional geometry of the human form.

Surface transformations in this context can involve various techniques to process and analyze depth images to extract useful features or parameters. One of the fundamental tasks is the extraction of the human body's skeletal structure or pose estimation. This process involves detecting key joints, such as the head, shoulders, elbows, wrists, hips, knees, and ankles, and estimating their positions in three-dimensional space.

Another important surface transformation is shape analysis, which aims to extract shape descriptors and characteristics from depth images. Shape analysis techniques can provide insights into body proportions, body segment lengths, and even detect anomalies or deviations from normal body structures. The information gathered by the surface transformation can comprise parameters describing the object under investigation based on the object information, e.g. a depth image.

According to an aspect of the invention, the surface transformation function is a rigid registration function or a non-rigid registration function.

Rigid transformations refer to transformations that preserve the shape and size of an object. These transformations include translation, rotation, and reflection. The transformation involves moving an object or shape without changing its orientation or size. It is characterized by shifting the object's position in a specific direction. The rotation involves rotating an object or shape around a fixed point, often referred to as the center of rotation. The object maintains its size and shape but is repositioned at a different angle. The reflection is a transformation that involves flipping or mirroring an object or shape across a line or plane. The object appears as a mirror image of itself but retains its shape and size.

Non-rigid transformations, also known as deformations, refer to transformations that can change the shape and size of an object. Unlike rigid transformations, non-rigid transformations can alter the local geometry of an object. These transformations are used to model objects that can undergo elastic deformations, such as the human body. Non-rigid transformations can comprise scaling, shearing, bending or warping.

The scaling is a non-rigid transformation that involves resizing an object or shape. It can either increase or decrease the size of an object uniformly or along different dimensions. The shearing is a non-rigid transformation that skews the shape of an object along one or more axes. It distorts the object by displacing points in a particular direction proportional to their distance from a reference line. Bending or warping involves nonlinear deformations that change the shape of an object in a localized manner. These transformations are used to model complex deformations in objects or surfaces.

In a next step, the examination pose is evaluated based on the adapted object model. The assessment of the positioning can be determined based on the matched avatar or the adapted object model. In this step, the results from the avatar-based simulation phase and adapting the object model to the object under investigation based on the object information can be combined. The object model parameters of the adapted object model are used as input for querying the database created by the avatar-based simulation phase and the statistical most likely measurement deviation is obtained. In case the measurement deviation exceeds a pre-defined threshold, the human operator can be informed about the deviation.

According to an aspect of the invention, the object model further relates to the object information. The object information can comprise a parameter like age, height, weight or other descriptive parameters of the object under investigation. The corresponding object model can be described by the same parameter with at least a similar value.

According to an aspect of the invention, the adapted object model is defined by object model parameters. The object model parameters can comprise a set of parameters describing the object model. The object model parameters can be used for the determination of the deviation. By querying the database based on the object model parameters, a deviation can be estimated or determined.

According to an aspect of the invention, a reference object model is selected based on the object model parameters from a plurality of reference object models with assigned positioning deviations compared to a standard examination pose. The reference object models can be stored in a database. Due to the finite nature of a database, the object model parameters can be used to select a reference object model out of a plurality of reference object models. A reference object model can be selected based on a comparison of object model parameters describing the adapted object model with corresponding parameters of the reference object models.

According to an aspect of the invention, an output is generated, if the positioning deviation of the selected reference object model exceeds a predefined threshold. The user can receive a message, if the positioning deviation exceeds a pre-defined threshold. An exceeded predefined threshold can correspond to malpositioning or a bad positioning of the object under investigation which would lead to a bad image for the imaging task or examination. The output can be displayed on a display. The output can be communicated to the user via optic, acoustic or haptic means. The amount of deviation can be made available to the user. Therefore, the user can decide whether a change of the position or pose of the object under investigation is needed or possible, especially regarding individual circumstances or individual parameters.

According to an aspect of the invention, the output is a proposal for an improved examination pose which is determined based on an individual parameter of the object under investigation. According to an aspect of the invention, the individual parameter comprises individual restrictions of mobility or individual restrictions due to pain associated with an examination pose. The individual parameter can describe an amount of pain, e.g. related to a movement of a joint or a fixation of a joint, e.g. by a plaster cast.

In a preferred embodiment, a recommendation of an alternative positioning is proposed to the user. When patient positioning has been evaluated to be suboptimal, especially a statistical measurement deviation exceeds a certain threshold, then a more suitable positioning of the object under investigation can be suggested by the system. This suggestion or this proposal can also include individual restrictions of the patient, e. g. due to physical disabilities.

Mathematically, the new suggested patient positioning can be described by the avatar parameters: ${p}^{sugg} = {\left[{{p}_{1}}^{sugg},{{p}_{2}}^{sugg},…\right]}^{T} .$ p₁^{sugg} could refer to a specific flexion of the knee and P₂^{sugg} could refer to a specific internal rotation of the knee, for example. The following relation must be fulfilled for a suggested new patient positioning: $D \left({p}^{sugg}\right) \leq {t}_{D}$ where D() is the measurement deviation depending on avatar parameters (see the step of adapting the object model to the object under investigation based on the object information) and t_{D} is a threshold value.

For individual patients, some patient positions may not be easily possible or may be painful , e.g. due to physical disabilities. Cₚₐₜ(**p**) is a function that describes the cost associated with a certain patient positioning for a certain patient (how it can be determined is described below). The cost is very high for positions that are not easily possible since they are painful.

The task is to find **p**^{sugg} such that $D \left({p}^{sugg}\right) \leq {t}_{D} and {C}_{pat} \left({p}^{sugg}\right) \leq {t}_{C}$ where t_{C} is the maximum tolerable cost. The task can be reformulated to minimize the following function $f \left({p}^{sugg}\right) = D \left({p}^{sugg}\right) - {b * C}_{pat} \left({p}^{sugg}\right)$ with chosen weighting b between measurement deviation and cost.

Standard methods for this optimization task exist. The function Cₚₐₜ(**p**) has to be determined individually for each patient. Thus, individual information about the patient is necessary to construct this function. The individual information could be obtained by using room camera that record the mobility of the patient, for example. Other sources from which Cₚₐₜ(**p**) can be determined are previous images or patient records. If patient-individual information is not available a statistical average from a population could be selected.

To sum up, the invention solves the problem using in a preferred embodiment the following steps:
- Step 0: Avatar-based simulation phase which can provide a set of reference object models as output;
- Step 1: Match avatar to patient which can comprise the steps of receiving an object information of the object under investigation in an examination pose, retrieving an object model relating to the examination pose and the region of interest, and adapting the object model to the object under investigation based on the object information,
- Step 2: Assessment based on matched avatar which can comprise evaluating the examination pose based on the adapted object model,
- Step 3 (optional): Recommendation of alternative positioning which can comprise a proposal for an improved ex-amination pose which is determined based on an individual parameter of the object under investigation.

Compared to a visual assessment by the radiographer or user which is current standard of care, the invention provides an automated assessment that is less subjective and more time efficient. As a unique feature, the painfulness of the patient positioning for certain patients is included in the determination of a more optimal patient positioning. The patient positioning can be assessed before X-ray radiation has been delivered to the patient which is an advantage and helps to work according to the ALARA principle to reduce the dose for the patient as much as possible. In case a pre-shot image is involved, only a low amount of X-ray radiation dose would be needed in order to assess the quality of the positioning.

The invention further relates to a computer-implemented method to create a set of reference object models for at least one examination pose and at least one region of interest, comprising:
- providing a plurality of different object models comprising at least one landmark for the corresponding region of interest,
- adapting the plurality of different object models to the at least one examination pose to generate reference object models,
- for each reference object model, generating a forward projection of the at least one landmark comprised by the reference object model in an X-ray geometry relating to the examination pose and the region of interest,
- providing the plurality of different object models in a standard examination pose,
- generating a forward projection of the at least one landmark comprised by the object model in a standard examination pose in the X-ray geometry relating to the examination pose and the region of interest, and
- determining a positioning deviation by comparing the forward projection of the reference object model to a forward projection of the corresponding object model in the standard examination pose.

A set of reference object models is created for at least one examination pose and at least one region of interest. The region of interest can be specified by an imaging task. The region of interest usually relates to a anatomical region of the object under investigation, e.g. thorax, shoulder or any other part of the human body.

A plurality of different object models comprising at least one landmark for the corresponding region of interest is provided. The plurality of different object models can be stored in a database. The plurality of different object models can be retrieved from the database.

The plurality of different object models is adapted to the at least one examination pose to generate reference object models. The at least one examination pose can deviate from a standard examination pose. A plurality of examination poses can provide a variety of deviations from a standard examination pose wherein the deviation can range from minor deviations to strong deviations.

For each reference object model, a forward projection of the at least one landmark comprised by the reference object model is generated in an X-ray geometry relating to the examination pose and the region of interest.

In addition, the plurality of different object models is provided in a standard examination pose. A forward projection of the at least one landmark comprised by the object model is generated in a standard examination pose in the X-ray geometry relating to the examination pose and the region of interest.

A positioning deviation is determined by comparing the forward projection of the reference object model to the forward projection of the corresponding object model in the standard examination pose.

In order to be able to use this method, at least one object model, preferably a plurality of object models, are made available. The inventors propose to create a number of reference object models based on an avatar-based simulation phase. This step can be required to be done only once per measurement application. Results from this step can be used in the steps of adapting the object model to the object under investigation, the assessment or evaluation based on the matched avatar or adapted object model, and/or a recommendation of alternative positioning based on data of individual patients or individual objects under investigation.

The aim of the avatar-based simulation phase is to explore the relationship between patient positioning as the patient is represented by an avatar or by the object model and its impact on the measurement result. The results of simulation phase provide a relationship between the positioning of the object under investigation, which can be represented by the object model, and the impact on the measurement result, especially based on an acquired X-ray image of the object under investigation which is positioned in that specific position and/or pose.

Preferably, different object models are made available. A specific object model can be selected from the plurality of object models. The plurality of object models can include object models of young, old, normal, obese, slim, small, large or medium sized patients. The object under investigation is preferably a human patient. Anatomical models of the relevant anatomy, e.g. based on CT volumes, can be used to refine the object model. In a preferred embodiment, a motion model for the object model can be required in addition to the anatomical model. The motion model can provide a model regarding realistic motion of bones. The object model includes at least one landmark, especially a plurality of relevant landmarks, in 3D for the corresponding measurement task or examination. In a preferred embodiment, the object model must include relevant landmarks in 3D for the corresponding measurement task or examination. The imaging process is modelled by a forward projector for the landmarks in X-ray geometry. The landmarks of the object model are projected in X-ray geometry into an imaging plane. The anatomical model as well as the motion model are used to modify the object model in order to provide a reference object model wherein the patient position parameters **p** can be used to characterize the reference object model and/or the object model.

For the measurement task, which could be indicated by index j, but is omitted here in most formulas for better readability, relevant patient position parameters **p** must be defined. These comprise the patient posture, the patient orientation relative to the imaging setup and the geometry of the imaging system.

A reference position **p**^{ref} needs to be defined: ${p}^{ref} = {\left[{{p}_{1}}^{ref},{{p}_{2}}^{ref}…\right]}^{T} .$

For this reference position, the projected 2D landmarks **l**^{ref} are obtained by applying the forward projection to the 3D landmarks **l**^{vol} of the object model at position **p**^{ref}. From **p**^{ref}, the reference measurement m^{ref} is calculated using the function m^{ref} = M^{j} (**p**^{ref}) where M^{j} () describes the measurement process for a particular measurement or examination. The reference position can characterize the pose and/or the position of the reference object model, especially in a standard examination pose. The position can characterize an examination pose which can be different to the standard examination pose. Equally, measurements mⁱ for positions **p**ⁱ covering the parameter space are obtained. The deviation ${d}^{i} = {m}^{ref} - {m}^{i} = M \left({p}^{ref}\right) - M \left({p}^{i}\right) = D \left({p}^{i}\right)$ of the measurements mⁱ to the reference measurement m^{ref} can be given as relative or absolute deviation. D() is a function depending on **p**ⁱ that computes the deviation for a pre-defined **p**^{ref}. The values dⁱ corresponding to **p**ⁱ are stored in a database. The values dⁱ can be given as absolute or relative values.

The invention further relates to a medical X-ray system comprising:
- a first interface, configured for receiving object information of the object under investigation in an examination pose,
- a second interface, configured for retrieving an object model relating to the examination pose and a region of interest,
- a third interface, configured for adapting the object model to the object under investigation based on the object information,
- a fourth interface, configured for evaluating the examination pose based on the adapted object model, wherein the adapted object model is defined by object model parameters, wherein a reference object model is selected based on the object model parameters from a plurality of reference object models with assigned positioning deviations compared to a standard examination pose.

The invention further relates to a computer program product comprising instructions which, when the program is executed by a computer, cause the computer to carry out the method according to the invention.

The invention further relates to a computer-readable medium comprising instructions which, when executed by a computer, cause the computer to carry out the method according to the invention.

Examples of embodiments of the invention are explained in more detail below by means of drawings.
FIG 1 a schematic representation of the method for a positioning control of an object under investigation before acquiring a projective X-ray image of a region of interest according to the invention;
FIG 2 a schematic representation of the method to create a set of reference object models for at least one examination pose and at least one region of interest according to the invention; and
FIG 3 a schematic representation of the medical system according to the invention.

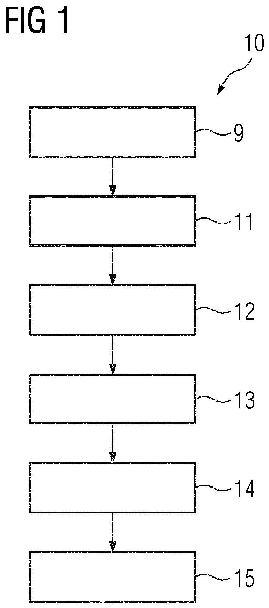

Fig. 1 shows an exemplary embodiment of a computer-implemented method 10 according to the invention. It is a method 10 for a positioning control of an object under investigation before acquiring a projective X-ray image of a region of interest, comprising:
- receiving 11 an object information of the object under investigation in an examination pose,
- retrieving 12 an object model relating to the examination pose and the region of interest,
- adapting 13 the object model to the object under investigation based on the object information,
- evaluating 14 the examination pose based on the adapted object model.

The computer-implemented method 10 according to the invention is used to control or to check a positioning of an object under investigation before acquiring a projective X-ray image of a region of interest. Typically, the object under investigation is a human patient.

The method 10 comprises the steps of receiving 11 , retrieving 12, adapting 13, and evaluating14, preferably in the given order. An object information of the object under investigation in an examination pose is received. An object model relating to the examination pose and the region of interest is retrieved, e.g. from a database. The object model is adapted to the object under investigation based on the object information. The examination pose is evaluated based on the adapted object model.

In order to be able to use this method 10, at least one object model, preferably a plurality of object models, are made available. The inventors propose to create a number of reference object models based on an avatar-based simulation phase. This step can be required to be done only once per measurement application. Results from this step can be used in the steps of adapting the object model to the object under investigation, the assessment or evaluation based on the matched avatar or adapted object model, and/or a recommendation of alternative positioning based on data of individual patients or individual objects under investigation.

The aim of the avatar-based simulation phase is to explore the relationship between patient positioning as the patient is represented by an avatar or by the object model and its impact on the measurement result. The results of simulation phase provide a relationship between the positioning of the object under investigation, which can be represented by the object model, and the impact on the measurement result, especially based on an acquired X-ray image of the object under investigation which is positioned in that specific position and/or pose.

Preferably, different object models are made available. A specific object model can be selected from the plurality of object models. The plurality of object models can include object models of young, old, normal, obese, slim, small, large or medium sized patients. The object under investigation is preferably a human patient. Anatomical models of the relevant anatomy, e.g. based on CT volumes, can be used to refine the object model. In a preferred embodiment, a motion model for the object model can be required in addition to the anatomical model. The motion model can provide a model regarding realistic motion of bones. The object model includes at least one landmark, especially a plurality of relevant landmarks, in 3D for the corresponding measurement task or examination. In a preferred embodiment, the object model must include relevant landmarks in 3D for the corresponding measurement task or examination. The imaging process is modelled by a forward projector for the landmarks in X-ray geometry. The landmarks of the object model are projected in X-ray geometry into an imaging plane. The anatomical model as well as the motion model are used to modify the object model in order to provide a reference object model wherein the patient position parameters **p** can be used to characterize the reference object model and/or the object model.

For the measurement task, which could be indicated by index j, but is omitted here in most formulas for better readability, relevant patient position parameters **p** must be defined. These comprise the patient posture, the patient orientation relative to the imaging setup and the geometry of the imaging system.

A reference position **p**^{ref} needs to be defined: ${p}^{ref} = {\left[{{p}_{1}}^{ref},{{p}_{2}}^{ref}…\right]}^{T} .$

For this reference position, the projected 2D landmarks **l**^{ref} are obtained by applying the forward projection to the 3D landmarks **l**^{vol} of the object model at position **p**^{ref}. From **p**^{ref}, the reference measurement m^{ref} is calculated using the function m^{ref} = M^{j} (**p**^{ref}) where M^{j} () describes the measurement process for a particular measurement or examination. The reference position can characterize the pose and/or the position of the reference object model, especially in a standard examination pose. The position can characterize an examination pose which can be different to the standard examination pose.

Equally, measurements mⁱ for positions **p**ⁱ covering the parameter space are obtained. The deviation ${d}^{i} = {m}^{ref} - {m}^{i} = M \left({p}^{ref}\right) - M \left({p}^{i}\right) = D \left({p}^{i}\right)$ of the measurements mⁱ to the reference measurement m^{ref} can be given as relative or absolute deviation. D() is a function depending on **p**ⁱ that computes the deviation for a pre-defined **p**^{ref}. The values dⁱ corresponding to **p**ⁱ are stored in a database. The values dⁱ can be given as absolute or relative values.

By receiving 11 an object information of the object under investigation in an examination pose, object information is provided e.g. based on information acquired by an external source or sensor, or the X-ray detector in combination with the X-ray source, especially a pre-shot X-ray image acquisition. The object information can be a depth image of the object under investigation. The object information can be determined based on an image of the object under investigation. The object under investigation is positioned preferably according to the examination pose in connection with the X-ray system. A specific examination pose is assigned to a specific X-ray examination. An examination pose can describe e.g. a standing, sitting or lying pose. The examination pose can include instructions for angles or rotations of body parts.

In a preferred embodiment, the object information is determined based on an image of the surface of the object under examination. The image of the surface can be acquired by a sensor or an external source. The external source or the sensor that records the external appearance of the patient, especially not recording the interior of the object under investigation, can include:
- an RGB camera,
- a depth camera,
- a structured light camera,
- an infrared camera, and/or
- a light detection and ranging (LiDAR) camera.

The external source or the sensor can be positioned at the medical system, at the imaging device or at a different location in the examination room.

The object information can be determined e.g. based on a landmark detection method. The object information can be determined e.g. based on a detection of the outline of the object under investigation. The object information can comprise a parameter relating to the size and/or weight of the object under investigation. The size can describe the full body length, or a measure of a body part or a body region. The object information can comprise the outer shape of the object under examination, especially the outer shape which can be observed from the position of the X-ray source. The outer shape can be made available in a grid representation comprising the depth information of at least a part of the object under examination.

In another embodiment, the object information is determined based on an X-ray image. The X-ray image can be a low dose X-ray pre-shot image. The low dose X-ray pre-shot image can show some information of the interior of the object under investigation but with less detail compared to a full X-ray image. An X-ray pre-shot could be used to acquire an image, in this case, the image comprises information of the internal appearance of body of the object under examination. The X-ray image can show details in a two-dimensional plane like the outline as well as some information about the internal structures of the object under investigation. The internal structures can comprise information about dense objects like bones, about soft tissue and/or about air volumes. The X-ray image provides two-dimensional information displayed by gray values.

By retrieving 12 an object model relating to the examination pose and the region of interest, the object model is selected according to the examination pose and the region of interest and the object model made available to the system.

In a next step, the avatar can be matched to object under investigation. This means that the object model can be adapted to the object under investigation based on the object information. The object model can be called an avatar. The object model can be adapted or modified based on the object information. The object information can comprise parameters like dimensions of anatomical structures or dimensions of the outer shape of the object under investigation.

Data from external sources can be used to adapt the parameters of the avatar to the real patient. The object information can be acquired by external sources like a camera. The object information can comprise parameters which describe at least the outer shape of the object under investigation, respectively the patient. The object information is used to adapt the object model to the object under investigation. Preferably, the object information comprises a set of parameters which describe the object under investigation as well as the object model. A parameter could be for example the width of the chest of the object under investigation and the object model, respectively. The object information is used to adapt the object model in a way that at least the outer shape of the object is represented by the adapted object model.

To match the object model to the object under investigation, one has to find the correct surface transformation between surface of the object model and the object under investigation. Various methods for this task have been proposed in literature and could be used for this task. The work by Shuai Lin, Yu-Kun Lai, Ralph R. Martin, Shiyao Jin, Zhi-Quan Cheng, Color-aware surface registration, Computers & Graphics, Volume 58, 2016, Pages 31-42, https://doi.org/10.1016/j.cag.2016.05.007*,* for example, introduced a novel color-aware registration method for rigid and non-rigid registration in which the inputs from depth cameras will provide initial alignment and deformable registration steps will improve it for having a surface with high quality textures. It is known that this step has a certain range of accuracy due to individual characteristics of the object under investigation. Nevertheless, one can use this step or this method to obtain a statistical estimation with a certain confidence range.

In a preferred embodiment, the object model is adapted by using a surface transformation function based on the object information. The surface or the outer shape of the object model can be adapted to the surface or the outer shape of the object under investigation. The object information can be used to bring the surface of the object model in conformity with the surface of the object under investigation, at least to a certain degree.

Surface transformations of a human shape based on depth images are well known. These transformations involve the analysis and manipulation of depth images to extract meaningful information about the shape and structure of human bodies. By understanding the surface properties of a human shape, a gesture recognition and a pose estimation can be performed based on (depth) images.

Depth images capture the distance information of objects in a scene, typically represented as a two-dimensional matrix of depth values. These depth values provide a measurement of the distance between the camera and the observed object's surface. For human bodies, depth images can offer valuable insights into the shape, posture, and movements of individuals, as they can capture the three-dimensional geometry of the human form.

Surface transformations in this context can involve various techniques to process and analyze depth images to extract useful features or parameters. One of the fundamental tasks is the extraction of the human body's skeletal structure or pose estimation. This process involves detecting key joints, such as the head, shoulders, elbows, wrists, hips, knees, and ankles, and estimating their positions in three-dimensional space.

Another important surface transformation is shape analysis, which aims to extract shape descriptors and characteristics from depth images. Shape analysis techniques can provide insights into body proportions, body segment lengths, and even detect anomalies or deviations from normal body structures. The information gathered by the surface transformation can comprise parameters describing the object under investigation based on the object information, e.g. a depth image.

In a preferred embodiment, the surface transformation function is a rigid registration function or a non-rigid registration function.

Rigid transformations refer to transformations that preserve the shape and size of an object. These transformations include translation, rotation, and reflection. The transformation involves moving an object or shape without changing its orientation or size. It is characterized by shifting the object's position in a specific direction. The rotation involves rotating an object or shape around a fixed point, often referred to as the center of rotation. The object maintains its size and shape but is repositioned at a different angle. The reflection is a transformation that involves flipping or mirroring an object or shape across a line or plane. The object appears as a mirror image of itself but retains its shape and size.

Non-rigid transformations, also known as deformations, refer to transformations that can change the shape and size of an object. Unlike rigid transformations, non-rigid transformations can alter the local geometry of an object. These transformations are used to model objects that can undergo elastic deformations, such as the human body. Non-rigid transformations can comprise scaling, shearing, bending or warping.

The scaling is a non-rigid transformation that involves resizing an object or shape. It can either increase or decrease the size of an object uniformly or along different dimensions. The shearing is a non-rigid transformation that skews the shape of an object along one or more axes. It distorts the object by displacing points in a particular direction proportional to their distance from a reference line. Bending or warping involves nonlinear deformations that change the shape of an object in a localized manner. These transformations are used to model complex deformations in objects or surfaces.

In a next step, the examination pose is evaluated based on the adapted object model. The assessment of the positioning can be determined based on the matched avatar or the adapted object model. In this step, the results from the avatar-based simulation phase and adapting the object model to the object under investigation based on the object information can be combined. The object model parameters of the adapted object model are used as input for querying the database created by the avatar-based simulation phase and the statistical most likely measurement deviation is obtained. In case the measurement deviation exceeds a pre-defined threshold, the human operator can be informed about the deviation.

In a preferred embodiment, the object model further relates to the object information. The object information can comprise a parameter like age, height, weight or other descriptive parameters of the object under investigation. The corresponding object model can be described by the same parameter with at least a similar value.

In a preferred embodiment, the adapted object model is defined by object model parameters. The object model parameters can comprise a set of parameters describing the object model. The object model parameters can be used for the determination of the deviation. By querying the database based on the object model parameters, a deviation can be estimated or determined.

In a preferred embodiment, a reference object model is selected based on the object model parameters from a plurality of reference object models with assigned positioning deviations compared to a standard examination pose. The reference object models can be stored in a database. Due to the finite nature of a database, the object model parameters can be used to select a reference object model out of a plurality of reference object models. A reference object model can be selected based on a comparison of object model parameters describing the adapted object model with corresponding parameters of the reference object models.

In a preferred embodiment, an output is generated, if the positioning deviation of the selected reference object model exceeds a predefined threshold. The user can receive a message, if the positioning deviation exceeds a predefined threshold. An exceeded predefined threshold can correspond to malpositioning or a bad positioning of the object under investigation which would lead to a bad image for the imaging task or examination. The output can be displayed on a display. The output can be communicated to the user via optic, acoustic or haptic means. The amount of deviation can be made available to the user. Therefore, the user can decide whether a change of the position or pose of the object under investigation is needed or possible, especially regarding individual circumstances or individual parameters.

According to an aspect of the invention, the output is a proposal 15 for an improved examination pose which is determined based on an individual parameter of the object under investigation. According to an aspect of the invention, the individual parameter comprises individual restrictions of mobility or individual restrictions due to pain associated with an examination pose. The individual parameter can describe an amount of pain, e.g. related to a movement of a joint or a fixation of a joint, e.g. by a plaster cast.

In a preferred embodiment, a recommendation of an alternative positioning is proposed to the user. When patient positioning has been evaluated to be suboptimal, especially a statistical measurement deviation exceeds a certain threshold, then a more suitable positioning of the object under investigation can be suggested by the system. This suggestion or this proposal can also include individual restrictions of the patient, e. g. due to physical disabilities.

Mathematically, the new suggested patient positioning can be described by the avatar parameters: ${p}^{sugg} = {\left[{{p}_{1}}^{sugg}, {{p}_{2}}^{sugg}, …\right]}^{T} .$ p₁^{sugg} could refer to a specific flexion of the knee and p₂^{sugg} could refer to a specific internal rotation of the knee, for example. The following relation must be fulfilled for a suggested new patient positioning: $D \left({p}^{sugg}\right) \leq {t}_{D}$ where D() is the measurement deviation depending on avatar parameters (see the step of adapting the object model to the object under investigation based on the object information) and t_{D} is a threshold value.

For individual patients, some patient positions may not be easily possible or may be painful , e.g. due to physical disabilities. Cₚₐₜ(**p**) is a function that describes the cost associated with a certain patient positioning for a certain patient (how it can be determined is described below). The cost is very high for positions that are not easily possible since they are painful.

The task is to find **p**^{sugg} such that $D \left({p}^{sugg}\right) \leq {t}_{D} and {C}_{pat} \left({p}^{sugg}\right) \leq {t}_{C}$ where t_{C} is the maximum tolerable cost. The task can be reformulated to minimize the following function $f \left({p}^{sugg}\right) = D \left({p}^{sugg}\right) - b * {C}_{pat} \left({p}^{sugg}\right)$ with chosen weighting b between measurement deviation and cost.

Standard methods for this optimization task exist. The function Cₚₐₜ(**p**) has to be determined individually for each patient. Thus, individual information about the patient is necessary to construct this function. The individual information could be obtained by using room camera that record the mobility of the patient, for example. Other sources from which Cₚₐₜ(**p**) can be determined are previous images or patient records. If patient-individual information is not available a statistical average from a population could be selected.

In step 9, the object information is determined based on an image of the surface of the object under examination. The object information can be represented by a depth or X-ray image of the object under investigation. The object information can be determined based on an X-ray image.

In step 13, the object model is adapted by using a surface transformation function based on the object information. The surface transformation function is a rigid registration function or a non-rigid registration function.

In step 12, the object model further relates to the object information, e.g. age or height. In step 13, the adapted object model is defined by object model parameters.

In step 14, a reference object model is selected based on the object model parameters from a plurality of reference object models with assigned positioning deviations compared to a standard examination pose. The object model can be stored in a database.

An output is generated, if the positioning deviation of the selected reference object model exceeds a predefined threshold.

In step 15, an output is generated and the output is a proposal for an improved examination pose which is determined based on individual parameters of the object under investigation. Individual parameters can comprise a parameter regarding pain. The individual parameters comprise individual restrictions of mobility or individual restrictions due to pain associated with an examination pose.

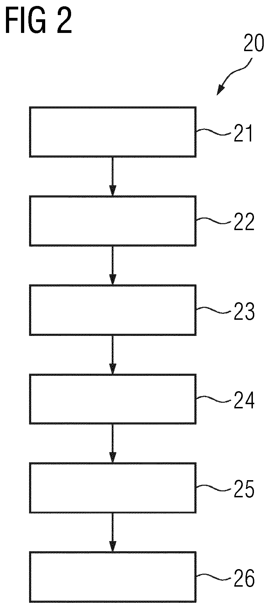

Fig. 2 shows an exemplary embodiment of a computer-implemented method 20 to create a set of reference object models for at least one examination pose and at least one region of interest, comprising:
- providing 21 a plurality of different object models comprising at least one landmark for the corresponding region of interest,
- adapting 22 the plurality of different object models to the at least one examination pose to generate reference object models,
- for each reference object model, generating 23 a forward projection of the at least one landmark comprised by the reference object model in an X-ray geometry relating to the examination pose and the region of interest,
- providing 24 the plurality of different object models in a standard examination pose,
- generating 25 a forward projection of the at least one landmark comprised by the object model in a standard examination pose in the X-ray geometry relating to the examination pose and the region of interest, and
- determining 26 a positioning deviation by comparing the forward projection of the reference object model to a forward projection of the corresponding object model in the standard examination pose.

A set of reference object models is created for at least one examination pose and at least one region of interest. The region of interest can be specified by an imaging task. The region of interest usually relates to a anatomical region of the object under investigation, e.g. thorax, shoulder or any other part of the human body.

A plurality of different object models comprising at least one landmark for the corresponding region of interest is provided in step 21. The plurality of different object models can be stored in a database. The plurality of different object models can be retrieved from the database.

In step 22, the plurality of different object models is adapted to the at least one examination pose to generate reference object models. The at least one examination pose can deviate from a standard examination pose. A plurality of examination poses can provide a variety of deviations from a standard examination pose wherein the deviation can range from minor deviations to strong deviations.

For each reference object model, a forward projection of the at least one landmark comprised by the reference object model is generated in an X-ray geometry relating to the examination pose and the region of interest in step 23.

In addition, the plurality of different object models is provided in a standard examination pose in step 24. A forward projection of the at least one landmark comprised by the object model is generated in a standard examination pose in the X-ray geometry relating to the examination pose and the region of interest in step 25.

In step 26, a positioning deviation is determined by comparing the forward projection of the reference object model to the forward projection of the corresponding object model in the standard examination pose.

In order to be able to use the method for a positioning control of an object under investigation before acquiring a projective X-ray image of a region of interest, at least one object model, preferably a plurality of object models, are made available. The inventors propose to create a number of reference object models based on an avatar-based simulation phase. This step can be required to be done only once per measurement application. Results from this step can be used in the steps of adapting the object model to the object under investigation, the assessment or evaluation based on the matched avatar or adapted object model, and/or a recommendation of alternative positioning based on data of individual patients or individual objects under investigation.

The aim of the avatar-based simulation phase is to explore the relationship between patient positioning as the patient is represented by an avatar or by the object model and its impact on the measurement result. The results of simulation phase provide a relationship between the positioning of the object under investigation, which can be represented by the object model, and the impact on the measurement result, especially based on an acquired X-ray image of the object under investigation which is positioned in that specific position and/or pose.

Preferably, different object models are made available. A specific object model can be selected from the plurality of object models. The plurality of object models can include object models of young, old, normal, obese, slim, small, large or medium sized patients. The object under investigation is preferably a human patient. Anatomical models of the relevant anatomy, e.g. based on CT volumes, can be used to refine the object model. In a preferred embodiment, a motion model for the object model can be required in addition to the anatomical model. The motion model can provide a model regarding realistic motion of bones. The object model includes at least one landmark, especially a plurality of relevant landmarks, in 3D for the corresponding measurement task or examination.

In a preferred embodiment, the object model must include relevant landmarks in 3D for the corresponding measurement task or examination. The imaging process is modelled by a forward projector for the landmarks in X-ray geometry. The landmarks of the object model are projected in X-ray geometry into an imaging plane. The anatomical model as well as the motion model are used to modify the object model in order to provide a reference object model wherein the patient position parameters **p** can be used to characterize the reference object model and/or the object model.

For the measurement task, which could be indicated by index j, but is omitted here in most formulas for better readability, relevant patient position parameters **p** must be defined. These comprise the patient posture, the patient orientation relative to the imaging setup and the geometry of the imaging system.

A reference position **p**^{ref} needs to be defined: ${p}^{ref} = {\left[{{p}_{1}}^{ref}, {{p}_{2}}^{ref} …\right]}^{T} .$

For this reference position, the projected 2D landmarks **l**^{ref} are obtained by applying the forward projection to the 3D landmarks **l**^{vol} of the object model at position **p**^{ref}. From **p**^{ref}, the reference measurement m^{ref} is calculated using the function m^{ref} = M^{j} (**p**^{ref}) where M^{j} () describes the measurement process for a particular measurement or examination. The reference position can characterize the pose and/or the position of the reference object model, especially in a standard examination pose. The position can characterize an examination pose which can be different to the standard examination pose.

Equally, measurements mⁱ for positions **p**ⁱ covering the parameter space are obtained. The deviation ${d}^{i} = {m}^{ref} - {m}^{i} = M \left({p}^{ref}\right) - M \left({p}^{i}\right) = D \left({p}^{i}\right)$ of the measurements mⁱ to the reference measurement m^{ref} can be given as relative or absolute deviation. D() is a function depending on **p**ⁱ that computes the deviation for a pre-defined **p**^{ref}. The values dⁱ corresponding to **p**ⁱ are stored in a database. The values dⁱ can be given as absolute or relative values.

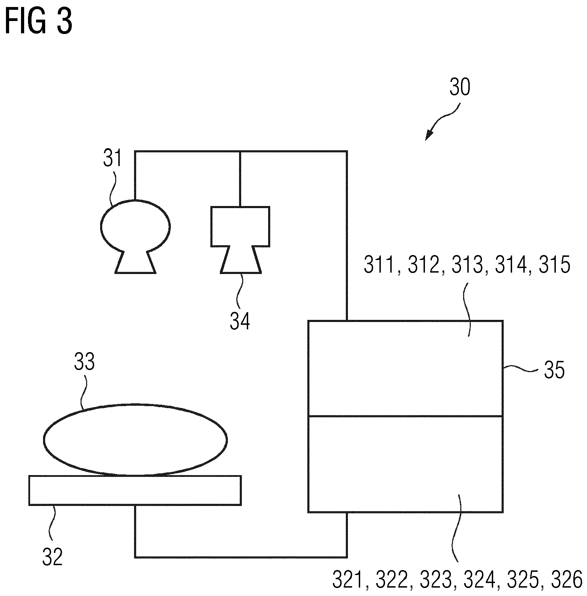

Fig. 3 shows an exemplary embodiment of a medical X-ray system comprising:
- a first interface 311, configured for receiving object information of the object under investigation in an examination pose,
- a second interface 312, configured for retrieving an object model relating to the examination pose and a region of interest,
- a third interface 313, configured for adapting the object model to the object under investigation based on the object information,
- a fourth interface 314, configured for evaluating the examination pose based on the adapted object model.

The interfaces 311 to 314 can be comprised by a computation unit 35. The computation unit 35 can further comprise units regarding the computer-implemented method to create a set of reference object models for at least one examination pose and at least one region of interest, comprising:
- a providing unit 321, configured for providing a plurality of different object models comprising at least one landmark for the corresponding region of interest,
- an adapting unit 322, configured for adapting the plurality of different object models to the at least one examination pose to generate reference object models,
- a generating unit 323, configured for generating a forward projection of the at least one landmark comprised by the reference object model in an X-ray geometry relating to the examination pose and the region of interest for each reference object model,
- a providing unit 324, configured for providing the plurality of different object models in a standard examination pose,
- a generating unit, configured for generating a forward projection of the at least one landmark comprised by the object model in a standard examination pose in the X-ray geometry relating to the examination pose and the region of interest, and
- a determining unit 326, configured for determining a positioning deviation by comparing the forward projection of the reference object model to a forward projection of the corresponding object model in the standard examination pose.

In an alternative embodiment, the units 321 to 326 are comprised by a separate computation unit.

In a preferred embodiment, the X-ray system 30 is a radiography system. In an alternative embodiment, the X-ray system 30 can be an angiography system, a fluoroscopy system, a mammography system, or a computed tomography system. The X-ray system 30 comprises an X-ray source 31 which emits X-rays towards the object under investigation 33. At least some of the X-rays can be registered by the X-ray detector 32. In a preferred embodiment, the external sensor is a (depth) camera 34. The camera 34, the X-ray source 31 and the X-ray detector 32 are connected to the computation unit 35.

Although the invention has been further illustrated in detail by the preferred embodiments, the invention is not limited by the disclosed examples and other variations may be derived therefrom by those skilled in the art without departing from the scope of protection of the invention.

## Claims

1. A computer-implemented method (10) for a positioning control of an object under investigation before acquiring a projective X-ray image of a region of interest, comprising:
- receiving (11) an object information of the object under investigation in an examination pose,
- retrieving (12) an object model relating to the examination pose and the region of interest,
- adapting (13) the object model to the object under investigation based on the object information,
- evaluating (14) the examination pose based on the adapted object model
**characterized in that**
the adapted object model is defined by object model parameters,
wherein a reference object model is selected based on the object model parameters from a plurality of reference object models with assigned positioning deviations compared to a standard examination pose.

2. Method according to claim 1, wherein the object information is determined based on an image of the surface of the object under examination.

3. Method according to one of the preceding claims, wherein the object information is determined based on an X-ray image.

4. Method according to one of the preceding claims, wherein the object model is adapted by using a surface transformation function based on the object information.

5. Method according to claim 4, wherein surface transformation function is a rigid registration function or a non-rigid registration function.

6. Method according to one of the preceding claims, wherein the object model further relates to the object information.

7. Method according to one of the preceding claims, wherein an output is generated, if the positioning deviation of the selected reference object model exceeds a predefined threshold.

8. Method according to claim 7, wherein the output is a proposal for an improved examination pose which is determined based on an individual parameter of the object under investigation.

9. Method according to claim 8, wherein the individual parameter comprises individual restrictions of mobility or individual restrictions due to pain associated with an examination pose.

10. A computer-implemented method (20) to create a set of reference object models for at least one examination pose and at least one region of interest, comprising:
- providing (21) a plurality of different object models comprising at least one landmark for the corresponding region of interest,
- adapting (22) the plurality of different object models to the at least one examination pose to generate reference object models,
- for each reference object model, generating (23) a forward projection of the at least one landmark comprised by the reference object model in an X-ray geometry relating to the examination pose and the region of interest,
- providing (24) the plurality of different object models in a standard examination pose,
- generating (25) a forward projection of the at least one landmark comprised by the object model in a standard examination pose in the X-ray geometry relating to the examination pose and the region of interest, and
- determining (26) a positioning deviation by comparing the forward projection of the reference object model to a forward projection of the corresponding object model in the standard examination pose.

11. A medical X-ray system (30) comprising:
- a first interface (311), configured for receiving object information of the object under investigation in an examination pose,
- a second interface (312), configured for retrieving an object model relating to the examination pose and a region of interest,
- a third interface (313), configured for adapting the object model to the object under investigation based on the object information,
- a fourth interface (314), configured for evaluating the examination pose based on the adapted object model, **characterized in that** the system is configured to perform the method of one of the claims 1 to 9.

12. A computer program product comprising instructions which, when the program is executed by a computer, cause the computer to carry out the method of one of the claims 1 to 9.

13. A computer-readable medium comprising instructions which, when executed by a computer, cause the computer to carry out the method of one of the claims 1 to 9.

## Patentansprüche

1. Computerimplementiertes Verfahren (10) für eine Positionssteuerung eines zu untersuchenden Objekts vor dem Erfassen eines projektiven Röntgenbildes einer interessierenden Region, umfassend:
- Empfangen (11) von Objektinformationen des zu untersuchenden Objekts in einer Untersuchungsstellung;
- Abrufen (12) eines Objektmodells, das sich auf die Untersuchungsstellung und die interessierende Region bezieht;
- Anpassen (13) des Objektmodells an das zu untersuchende Objekt basierend auf den Objektinformationen;
- Auswerten (14) der Untersuchungsstellung basierend auf dem angepassten Objektmodell
**dadurch gekennzeichnet, dass**
das angepasste Objektmodell durch Objektmodellparameter definiert wird,
wobei ein Referenzobjektmodell basierend auf den Objektmodellparametern aus einer Vielzahl von Referenzobjektmodellen mit zugewiesenen Positionsabweichungen im Vergleich zu einer Standarduntersuchungsstellung ausgewählt wird.

2. Verfahren nach Anspruch 1, wobei die Objektinformationen basierend auf einem Bild der Oberfläche des zu untersuchenden Objekts bestimmt werden.

3. Verfahren nach einem der vorhergehenden Ansprüche, wobei die Objektinformationen basierend auf einem Röntgenbild bestimmt werden.

4. Verfahren nach einem der vorhergehenden Ansprüche, wobei das Objektmodell unter Verwendung einer Oberflächentransformationsfunktion basierend auf den Objektinformationen angepasst wird.

5. Verfahren nach Anspruch 4, wobei die Oberflächentransformationsfunktion eine starre Registrierungsfunktion oder eine nicht-starre Registrierungsfunktion ist.

6. Verfahren nach einem der vorhergehenden Ansprüche, wobei sich das Objektmodell ferner auf die Objektinformationen bezieht.

7. Verfahren nach einem der vorhergehenden Ansprüche, wobei eine Ausgabe erzeugt wird, wenn die Positionsabweichung des ausgewählten Referenzobjektmodells eine vordefinierte Schwelle überschreitet.

8. Verfahren nach Anspruch 7, wobei die Ausgabe ein Vorschlag für eine verbesserte Untersuchungsstellung ist, die basierend auf einem individuellen Parameter des zu untersuchenden Objekts bestimmt wird.

9. Verfahren nach Anspruch 8, wobei der individuelle Parameter individuelle Mobilitätseinschränkungen oder individuelle Einschränkungen aufgrund von Schmerzen im Zusammenhang mit einer Untersuchungsstellung umfasst.

10. Computerimplementiertes Verfahren (20) zum Erzeugen eines Satzes von Referenzobjektmodellen für mindestens eine Untersuchungsstellung und mindestens eine interessierende Region, umfassend:
- Bereitstellen (21) einer Vielzahl von verschiedenen Objektmodellen, die mindestens einen Orientierungspunkt für die entsprechende interessierende Region umfassen,
- Anpassen (22) der Vielzahl von verschiedenen Objektmodellen an die mindestens eine Untersuchungsstellung, um Referenzobjektmodelle zu erzeugen,
- für jedes Referenzobjektmodell Erzeugen (23) einer Vorwärtsprojektion des mindestens einen Orientierungspunktes, der durch das Referenzobjektmodell in einer Röntgengeometrie umfasst ist, die sich auf die Untersuchungsstellung und die interessierende Region bezieht,
- Bereitstellen (24) der Vielzahl von verschiedenen Objektmodellen in einer Standarduntersuchungsstellung,
- Erzeugen (25) einer Vorwärtsprojektion des mindestens einen Orientierungspunktes, der durch das Objektmodell in einer Standarduntersuchungsstellung in der Röntgengeometrie umfasst ist, die sich auf die Untersuchungsstellung und die interessierende Region bezieht, und
- Bestimmen (26) einer Positionsabweichung durch Vergleichen der Vorwärtsprojektion des Referenzobjektmodells mit einer Vorwärtsprojektion des entsprechenden Objektmodells in der Standarduntersuchungsstellung.

11. Medizinisches Röntgensystem (30), umfassend:
- eine erste Schnittstelle (311), die zum Empfangen von Objektinformationen des zu untersuchenden Objekts in einer Untersuchungsstellung ausgelegt ist,
- eine zweite Schnittstelle (312), die zum Abrufen eines Objektmodells in Bezug auf die Untersuchungsstellung und eine interessierende Region ausgelegt ist,
- eine dritte Schnittstelle (313), die zum Anpassen des Objektmodells an das zu untersuchende Objekt basierend auf den Objektinformationen ausgelegt ist,
- eine vierte Schnittstelle (314), die zum Bewerten der Untersuchungsstellung basierend auf dem angepassten Objektmodell ausgelegt ist, **dadurch gekennzeichnet, dass** das System dazu ausgelegt ist, das Verfahren nach einem der Ansprüche 1 bis 9 durchzuführen.

12. Computerprogrammprodukt, umfassend Anweisungen,
die bei Ausführung des Programms durch einen Computer den Computer veranlassen, das Verfahren nach einem der Ansprüche 1 bis 9 durchzuführen.

13. Computerlesbares Medium, umfassend Anweisungen,
die bei Ausführung durch einen Computer den Computer veranlassen, das Verfahren nach einem der Ansprüche 1 bis 9 auszuführen.

## Revendications

1. Un procédé (10) mis en œuvre par ordinateur de commande de la mise en position d'un objet en examen avant d'acquérir une image projective aux rayons X d'une région à laquelle on s'intéresse, comprenant :
- recevoir (11) une information d'objet de l'objet en examen dans une pose d'examen,
- rechercher (12) un modèle d'objet se rapportant à la pose d'examen et à la région à laquelle on s'intéresse,
- adapter (13) le modèle d'objet pour l'objet en examen sur la base de l'information d'objet,
- évaluer (14) la pose d'examen sur la base du modèle d'objet adapté,
**caractérisé en ce que**
le modèle d'objet adapté est défini par des paramètres de modèle d'objet,
dans lequel un modèle d'objet de référence est choisi sur la base des paramètres de modèle d'objet parmi une pluralité de modèles d'objet de référence ayant des écarts de mise en position assignés par rapport à une pose d'examen standard.

2. Procédé suivant la revendication 1, dans lequel l'information d'objet est déterminée sur la base d'une image de la surface de l'objet en examen.

3. Procédé suivant l'une des revendications précédentes, dans lequel l'information d'objet est déterminée sur la base d'une image aux rayons X.

4. Procédé suivant l'une des revendications précédentes, dans lequel le modèle d'objet est adapté en utilisant une fonction de transformation de surface sur la base de l'information d'objet.

5. Procédé suivant la revendication 4, dans lequel la fonction de transformation de surface est une fonction d'alignement rigide ou une fonction d'alignement non rigide.

6. Procédé suivant l'une des revendications précédentes, dans lequel le modèle d'objet se rapporte en outre à l'information d'objet.

7. Procédé suivant l'une des revendications précédentes, dans lequel une sortie est créée, si l'écart de mise en position du modèle d'objet de référence sélectionné dépasse un seuil défini à l'avance.

8. Procédé suivant la revendication 7, dans lequel la sortie est une proposition d'une pose d'examen améliorée, qui est déterminée sur la base d'un paramètre individuel de l'objet en examen.

9. Procédé suivant la revendication 8, dans lequel le paramètre individuel comprend des restrictions individuelles de mobilité ou des restrictions individuelles dues à la douleur associée à une pose d'examen.

10. Un procédé (20) mis en œuvre par ordinateur pour créer un ensemble de modèles d'objet de référence pour au moins une pose d'examen et au moins une région à laquelle on s'intéresse, comprenant :
- se procurer (21) une pluralité de modèles d'objet différents comprenant au moins un repère pour la région correspondante à laquelle on s'intéresse,
- adapter (22) la pluralité de modèles d'objet différents à la au moins une pose d'examen pour créer des modèles d'objet de référence,
- pour chaque modèle d'objet de référence, créer (23) une projection vers l'avant du au moins un repère compris par le modèle d'objet de référence dans une géométrie de rayons X se rapportant à la pose d'examen et à la région à laquelle on s'intéresse,
- se procurer (24) la pluralité de modèles d'objet différents dans une pose d'examen standard,
- créer (25) dans une projection vers l'avant du au moins un repère compris par le modèle d'objet dans une pose d'examen standard dans la géométrie de rayons X se rapportant à la pose d'examen et à la région à laquelle on s'intéresse, et
- déterminer (26) un écart de mise en position en comparant la projection vers l'avant du modèle d'objet de référence à une projection vers l'avant du modèle d'objet correspondant dans la pose d'examen standard.

11. Un système (30) médical aux rayons X comprenant :
- une première interface (311), configurée pour recevoir une information d'objet de l'objet en examen dans une pose d'examen,
- une deuxième interface (312), configurée pour rechercher un modèle d'objet se rapportant à la pose d'examen et à la région à laquelle on s'intéresse,
- une troisième interface (313), configurée pour adapter le modèle d'objet à l'objet en examen sur la base de l'information d'objet,
- une quatrième interface (314), configurée pour évaluer la pose d'examen sur la base du modèle d'objet adapté,
**caractérisé en ce que** le système est configuré pour effectuer le procédé de l'une des revendications 1 à 9.

12. Un produit de programme d'ordinateur comprenant des instructions qui, lorsque le programme est exécuté par ordinateur, font que l'ordinateur effectue le procédé de l'une des revendications 1 à 9.

13. Un support déchiffrable par ordinateur comprenant des instructions qui, lorsqu'elles sont exécutées par un ordinateur, font que l'ordinateur effectue le procédé de l'une des revendications 1 à 9.
